(19) Europäisches Patentamt
European Patent Office
Office européen des brevets

(11) EP 0 740 957 A1

(12) DEMANDE DE BREVET EUROPEEN

(43) Date de publication:
**06.11.1996 Bulletin 1996/45**

(51) Int Cl.[6]: **B01J 29/06**, C07C 5/27

(21) Numéro de dépôt: **96400924.5**

(22) Date de dépôt: **30.04.1996**

(84) Etats contractants désignés:
**DE FR GB IT**

(30) Priorité: **04.05.1995 FR 9505463**

(71) Demandeur: **INSTITUT FRANCAIS DU PETROLE**
**92502 Rueil-Malmaison (FR)**

(72) Inventeurs:
- **Benazzi, Eric**
  **78360 Montesson (FR)**
- **Guisnet, Michel**
  **Buxerolles France (FR)**
- **Andy, Patricia**
  **Poitiers (FR)**
- **Travers, Christine**
  **92500 Rueil Malmaison (FR)**
- **Gnep, Ngi Suor**
  **86800 Bignoux (FR)**

(54) **Procédé d'isomerisation squelettale des olefines lineaires avec un tamis moléculaire prétraité et catalyseur contenant un tamis prétraité**

(57) L'invention concerne un procédé d'isomérisation squelettale des oléfines linéaires avec un catalyseur contenant au moins un tamis moléculaire prétraité choisi dans le groupe formé par sapo-31, SAPO-11, Theta-1, EU-1, zéolite oméga, mordénite, Nu-10, Nu-86, Nu-87, ferrierite, ZSM-12 et ZSM-23.

Le procédé de prétraitement consiste à mettre au contact le tamis moléculaire présentant une taille de pores de 0,4 à 0,8 nm, avec au moins une molécule hydrocarbonée ayant de 4 à 20 atomes de carbone, à une vitesse spatiale de 0,1-45$h^{-1}$, une température de 300-550 °C, une pression de 0,1-1 Mpa, pendant 0,5-48 h. de façon à déposer du coke dans lesdits pores. Le procédé s'applique particulièrement bien aux tamis monodimensionels. Il est de préférence effectué en dehors de la zone de réaction.

L'invention concerne également un catalyseur contenant un tamis ainsi prétraité, le tamis étant choisi parmi SAPO-31, SAPO-11, Theta-1, EU-1, zéolite oméga, mordénite, ferrierite, Nu-10, Nu-86 et Nu-87.

EP 0 740 957 A1

## Description

L'invention concerne un procédé d'isomérisation squelettale d'oléfines linéaires utilisant un tamis présentant une taille de pores comprise entre 0,4 et 0,8 nm et ayant subi un procédé de prétraitement sélectivant par cokage.

L'invention concerne également un catalyseur contenant un tamis choisi choisi dans le groupe formé par les SAPO-31, SAPO-11, Theta-1, EU-1, zéolite oméga, mordénite, ferrierite, Nu-10, Nu-86 et Nu-87 et qui a subi un traitement sélectivant par cokage

Des procédés de traitement par cokage ont déjà été décrits pour des catalyseurs utilisés en alkylation ou isomérisation d'aromatiques ou encore en oligomérisation d'oléfines.

Ainsi, le brevet US-4,508,836 décrit un procédé de traitement appliqué à un catalyseur utilisé pour convertir des charges aromatiques par alkylation ou isomérisation, ce catalyseur contenant une zéolite d'indice de contrainte compris entre 1 et 12 et en particulier une ZSM-5,-11,-12, -35, ou -38. Le traitement décrit consiste à mettre le catalyseur au contact d'un composé aromatique (toluène), éventuellement en présence d'hydrogène, à une température inférieure à 650°C, pour déposer plus de 1 % de coke.

Un autre brevet US-5,234,875 décrit un catalyseur utilisé pour l'oligomérisation d'oléfines, qui contient notamment une ZSM-23, et qui a été précoké avec une oléfine, à une température de 200-500°C et sous une pression supérieure à 27 bars.

Dans la présente invention, il est absolument essentiel d'éviter l'oligomérisation des oléfines (c'est-à-dire la polymérisation) qui diminuerait le rendement.

La réduction des alkyles de plomb dans les essences a conduit le raffineur à envisager depuis quelques années, l'incorporation à l'essence de différents composés et en particulier, des alcools et des éthers, permettant d'augmenter l'indice d'octane. Outre le méthanol qui est l'un des plus intéressants additifs connu, le MTBE (méthyl-tertio-butyl éther), possède des propriétés antidétonantes permettant l'amélioration de la qualité des essences et l'augmentation de leur indice d'octane, augmentation supérieure à celle obtenue avec le méthanol. Le MTBE possède également de nombreux autres avantages tels que :

- un point d'ébullition correspondant à celui des composants de l'essence qui présentent les plus faibles propriétés antidétonantes,
- une tension de vapeur compatible avec les composants précités,
- un excellent point de congélation,
- une solubilité dans l'eau faible,
- une miscibilité complète avec les hydrocarbures etc.

Le MTBE est généralement obtenu à partir d'isobutène et de méthanol d'après la réaction suivante :

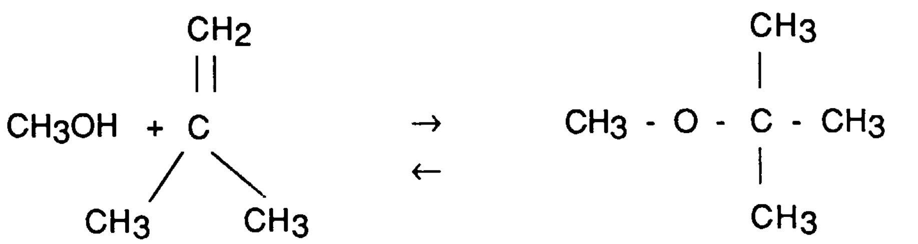

L'isobutène est généralement contenu dans les coupes oléfiniques $C_3$-$C_4$, issues des effluents du craquage catalytique, du vapocraquage, du craquage thermique et du procédé de viscoréduction. Cependant les quantités d'isobutène fournies par ces différents procédés ne sont pas suffisantes pour permettre un large développement du procédé de production de MTBE.

C'est pourquoi, il a été proposé, afin de produire de plus grandes quantités d'isobutène, d'isomériser complètement ou presque, les butènes contenus dans les effluents des procédés cités ci-dessus en isobutènes.

De nombreux procédés associés à de nombreux catalyseurs ont été proposés dans la littérature.

Les catalyseurs utilisés sont généralement à base d'alumine ou bien de tamis moléculaires.

De nombreux catalyseurs d'isomérisation des oléfines, plus précisément du butène en isobutène, incorporent dans leur formulation des tamis moléculaires possédant des réseaux microporeux bi-ou tridimensionnels dont les canaux sont interconnectés. Ces tamis peuvent être associés à une fonction métallique "hydrogénante" telle que le platine, le palladium ou le gallium. Les gros inconvénients de ce type de catalyseur sont :

- une désactivation dûe à la formation d'une importante quantité de coke,
- la formation de produits indésirables tels que des dimères et trimères des butènes ainsi que des aromatiques.

Le terme tamis moléculaire désigne les zéolithes c'est-à-dire les aluminosilicates microporeux cristallisés, synthétiques ou naturels, mais aussi d'autres tamis moléculaires tels que les silicoaluminophosphates SAPO (USP 4440871), les aluminophosphates et leurs dérivés intégrant un métal c'est-à-dire les MeAPO et ELAPO ainsi que les silicoaluminophosphates intégrant un métal c'est-à-dire les MeAPSO, ELAPSO.

Plus récemment, il a été montré que des zéolithes ou tamis moléculaire possédant un réseau microporeux monodimensionel dont l'ouverture de pores est supérieure à 0,42 nm et inférieur à 0,7 nm, (brevets EP-A-523838, EP-A-501577) peuvent constituer les phases actives de catalyseurs d'isomérisation squelettale des oléfines linéaires.

Les structures qui sont citées dans les brevets mentionnés ci-dessus sont la ferriédte, la SAPO-11 et une mordénite échangée au magnésium.

Lors de recherches visant à améliorer les performances de ces catalyseurs, il a été découvert de façon surprenante qu'un tamis moléculaire possédant de préférence un réseau microporeux monodimensionnel, tamis dont la taille de pores est comprise entre 0,4 et 0,8 nm, auquel on fait subir dans des conditions précises un prétraitement cokant permettant de réaliser un bouchage partiel voire presque total du volume microporeux, conduit à une sélectivité améliorée en iso-oléfine (isobutène par exemple) lors de l'isomérisation squelettale des oléfines des (n-butènes par exemple). Ces catalyseurs montrent également de bonnes stabilités catalytiques.

Un objet de l'invention est un procédé d'isomérisation squelettale avec un catalyseur contenant au moins un tamis moléculaire présentant une taille de pores comprise entre 0,4 nm et 0,8 nm, dans lequel ledit tamis a été préalablement mis au contact d'au moins un molécule hydrocarbonée ayant de 4 à 20 atomes de carbone, à une vitesse spatiale de 0,1-45$h^{-1}$, une température de 300-550°C, sous une pression de 0,1-1MPa, pendant 0,5-48h, de façon à déposer du coke dans lesdits pores dudit tamis.

La présente invention concerne avantageusement des tamis moléculaires dont la taille de pore est comprise entre 0,4 nm et 0,7 nm, et qui de préférence possèdent un réseau microporeux monodimensionnel. Parmi les tamis moléculaires qui peuvent être traités selon le procédé conforme à l'invention, on peut citer à titre d'exemples non limitatifs et non exhaustifs les structures suivantes; la Ferrierite (de type structural FER), le SAPO-31, le SAPO-11 (de type structural AEL), Theta-1 (de type structural TON), EU-1 (de type structural EUO), ZSM-12 (de type structural MTW), la ZSM-23 (de type structural MTT), la zéolite oméga, la mordénite, Nu-10, Nu-86 et Nu-87.

Le tamis moléculaire est traité avant (préféré) ou après mise en forme dans une matrice choisie dans le groupe formé de préférence par l'alumine, la magnésie, la silice alumine, les argiles naturelles (kaolin, bentonite) et par des techniques telles que l'extrusion, le pastillage ou la dragéification. Toute matrice connue de l'homme du métier est susceptible de convenir.

Le tamis peut également être utilisé tel que, sans matrice.

Des éléments des divers groupes de la classification périodique peuvent éventuellement avoir été introduits.

Le prétraitement cokant du tamis moléculaire mise en forme avec un liant ou non est réalisé par introduction d'au moins une molécule hydrocarbonée ayant 4 à 20 atomes de carbone et de préférence 4 à 12, de préférence choisie dans le groupe formé par les monooléfines, les polyoléfines ou les alcanes, et de préférence les alcanes ayant 4 à 12 atomes de carbone. La taille de cette molécule lui permet de pénétrer à l'intérieur de la microporosité du tamis.

Le prétraitement peut également s'effectuer par introduction de la charge à convertir si celle-ci contient la(les) molécule(s) citée(s). Ce prétraitement peut s'effectuer alors avantageusement avant le démarrage proprement dit de la réaction de conversion.

C'est le cas pour l'isomérisation des oléfines linéaires.

Le prétraitement a lieu préalablement à la réaction d'isomérisation, c'est-à-dire par exemple sur le tamis seul qui est le catalyseur, ou sur le tamis seul avant formation du catalyseur, ou sur le catalyseur contenant le tamis.

Le procédé a lieu à une vitesse spatiale comprise entre 0,1 et 45 h-1 et de préférence entre 0,5 et 25 h-1 et de manière encore plus préférée entre 0,5 et 10 h-1, à une température comprise entre 300 et 550°C et de préférence entre 400 et 550 °C et avantageusement plus de 400°C à 535°C, pendant une durée comprise entre 0,5 h et 48 h, de préférence entre 0,5 h et 24 h, sous une pression de 0,1-1 MPa et plus avantageusement de 0,1-0,5 MPa.

Avantageusement, la charge utilisée pour le prétraitement, et contenant au moins une molécule hydrocarbonée de 4 à 20 atomes de carbone, est diluée par exemple dans un gaz inerte (azote etc.). Le procédé se déroule en l'absence d'hydrogène.

Après ce traitement, la teneur en coke du tamis moléculaire est telle que le volume poreux accessible à l'azote et mesuré par adsorption d'azote, est compris entre 3 et 30 % du volume poreux du tamis de départ non coké, de préférence entre 5 et 20% et de manière encore plus préférée entre 5 et 15% du volume poreux du tamis moléculaire de départ non coké. Ce test montre que le cokage a lieu dans les pores et pas uniquement sur la surface externe.

Ce prétraitement conduit à une augmentation considérable de la sélectivité du catalyseur en particulier pour l'isomérisation squelettale des oléfines. Cette augmentation de sélectivité provient d'une diminution très marquée des

rendements en produits issus de la dismutation de l'oléfine de la charge ainsi que des produits paraffiniques qui résultent d'un transfert d'hydrogène des précurseurs de coke au réactif. Par ailleurs, l'activité du tamis moléculaire est peu modifiée par le prétraitement selon l'invention.

Ce procédé permet également d'obtenir des performances améliorées en particulier au niveau et de la stabilité du tamis et/ou du catalyseur.

Le procédé ici décrit peut être effectué de préférence à l'intérieur de la zone de réaction, dans une pré-zone de réaction au mieux dans une installation particulière dotée des équipements nécessaires. Il peut également être mis en oeuvre dans la zone de réaction.

Le procédé pour l'isomérisation squelettale d'une charge contenant des hydrocarbures oléfiniques linéaires ayant de 4 à 20 atomes de carbone, dans lequel la charge est mise au contact du catalyseur à une température de 150-500°C, une pression de 0,01-1 MPa, une vitesse spatiale de 0,1-10h-1, lesdit catalyseur comprenant un tamis moléculaire prétraité selon le procédé précédemment décrit.

La charge à isomériser est mise au contact du catalyseur à une température comprise entre 150°C et 500°C et de préférence entre 150-450°C en particulier lorsqu'elle est constituée de butènes et/ou de pentènes) à une pression comprise entre 0,01 et 1 MPa absolus (et préférentiellement entre 0,01 et 0,5 MPa absolus en particulier lorsque la charge est constituée de butènes et/ou de pentènes). La vitesse spatiale est comprise entre 0,1 et 10 $h^{-1}$ exprimée en volume de charge oléfinique par volume de catalyseur et par heure (et de manière préférée entre 0,5 et 6 $h^{-1}$ notamment lorsque la charge est constituée de butènes et/ou de pentènes).

Dans le procédé suivant l'invention, il est possible d'isomériser une coupe $C_4$ oléfinique seule (après avoir enlevé la coupe $C_3$), la totalité de la coupe $C_3$-$C_4$ oléfinique, une coupe $C_5$ oléfinique ou plus généralement des hydrocarbures oléfiniques linéaires contenant de 4 à 20 atomes de carbone par molécule, c'est-à-dire des coupes contenant majoritairement lesdits hydrocarbures.

Le catalyseur contient de 5-100 % de tamis et de préférence 10-90 % poids de tamis, et avantageusement 20-80 %, la matrice étant de préférence l'alumine.

L'invention concerne également un catalyseur contenant au moins un tamis moléculaire choisi dans le groupe formé par SAPO-31, SAPO-11, Theta-1, EU-1, la zéolite oméga, la mordénite, ferrierite, Nu-10, Nu-86 et Nu-87, ledit tamis ayant été soumis au traitement sélectivant par cokage précédemment décrit.

Ce traitement est de préférence conduit sous une pression de 0,1-0,5 MPa, entre 400 et 535°C, en l'absence d'hydrogène et en utilisant au moins une oléfine, une polyoléfine ou une alcane ayant de 4 à 12 atomes de carbone.

Les exemples suivants illustrent l'invention sans en limiter la portée.

Les performances en isomérisation sont exprimées par :

1/ la conversion des butènes

$$C=\frac{S\ (\%n\ \text{butènes})\ \text{charge} - S\ (\%n\ \text{butènes})\ \text{effluent}}{S\ (\%\ n\text{-butènes})\ \text{charge}}\times 100$$

2/ la sélectivité en isobutène

$$S=\frac{(\%\ \text{isobutène})\ \text{effluent} - (\%\ \text{isobutène})\ \text{charge}}{S\ (\%\ n\text{-butènes})\ \text{charge} - S\ (\%\ n\text{-butènes})\ \text{effluent}}\times 100$$

3/ le rendement en isobutène

$$R = C \times S/100$$

**Exemple 1 : Catalyseur 1 conforme à l'invention**

10g de ferrierite de taille de pores 0,42 nm x 0,54 nm et 0,35 nm x 0,48 nm, synthétisée selon le mode opératoire décrit dans les brevets US-4,853,203 et US-4,982,046 qui a été mise sous forme acide et dont le rapport Si/Al atomique est de 13,8 sont pastillés, puis tamisés pour obtenir une fraction dont le diamètre est compris entre 0,2 et 0,5 mm, puis 5g de ferrrierite pastillée et tamisée sont introduits dans un réacteur tubulaire afin d'être mis en oeuvre en lit fixe.

Cette ferrierite subit alors un alors une calcination sous air sec durant 4 heures à 550°C puis un prétraitement cokant lui est appliqué. Ce prétraitement consiste à réaliser une introduction de n-butènes (qui constituent également la charge à isomériser) à une température de 500°C durant 2 heures. Les n-butènes sont introduits dilués dans l'azote aux pressions partielles suivantes $P_{n\text{-butènes}}$ = 0,2 bar et $P_{N2}$ = 0,8 bar avec une vitesse spatiale de 2 grammes de n-butènes par gramme de ferrierite et par heure (2h-1).

Puis la température du réacteur est abaissée à 350°C, qui est la température de la réaction d'isomérisation des n-butènes et la pph maintenue à 2 h-1.

La conversion des n-butènes, les sélectivités et rendements en isobutène sont alors mesurés au bout de 1 heure d'injection de n-butènes et sont reportés dans le tableau 1.

On a pu vérifier des résultats similaires sur un catalyseur constitué de 80% pds de la zéolite utilisée dans cet exemple et de 20% pds d'alumine.

**Exemple 2 : Catalyseur 2 non conforme à l'invention**

La même ferrierite que celle utilisée dans l'exemple 1 est mise en oeuvre de la même façon dans réacteur à lit fixe et calcinée à 550°C durant 4 heures.

Puis, elle subit un prétraitement consiste à réaliser une introduction des n-butènes à isomériser à une température de 120°C durant 2 heures. Les n-butènes sont introduits dilués dans l'azote aux pressions partielles suivantes $P_{n\text{-butènes}}$ = 0,2 bar et $P_{N2}$ = 0,8 bar avec une vitesse spatiale de 2g de n-butènes par g de ferrierite et par heure (2h-1).

Puis la température du réacteur est augmentée à 350°C, qui est la température de la réaction d'isomérisation des n-butènes et la pph maintenue à 2 h-1.

La conversion des n-butènes, les sélectivités et rendements en isobutène sont alors mesurés au bout de 1 heure d'injection de n-butènes et sont reportés dans le tableau 1.

Tableau 1

| | Conversion n-butènes (% poids) | Sélectivité en isobutène (% poids) | Rendement en isobutène (% poids) |
|---|---|---|---|
| Exemple 1 conforme à l'invention | 45 | 90 | 40,5 |
| Exemple 2 non conforme à l'invention | 50 | 60 | 30 |

Ce tableau met bien en évidence l'influence du prétraitement selon l'invention de la Ferrierite sur les sélectivités et les rendements en isobutène qui sont dans ce cas plus élevés que lorsqu'aucun pétraitement, selon l'invention, n'est appliqué.

**Exemple 3 : Catalyseur 3 conforme à l'invention**

10g de zéolithe ZSM-23 de type structural MTT, synthétisée selon le mode opératoire décrit dans le brevet EP-A-347273, qui a été mise sous forme acide et dont le rapport Si/Al atomique est de 50 sont pastillés, puis tamisés pour obtenir une fraction dont le diamètre est compris entre 0,2 et 0,5 mm, puis 5g de ZSM-23 pastillée et tamisée sont introduits dans un réacteur tubulaire afin d'être mis en oeuvre en lit fixe.

Cette zéolithe ZSM-23 subit alors une calcination sous air sec durant 4 heures à 550°C puis un prétraitement cokant lui est appliqué. Ce prétraitement consiste à réaliser une introduction des n-butènes à isomériser à une température de 500°C durant 2 heures. Les n-butènes sont introduits dilués dans l'azote aux pressions partielles suivantes $P_{n\text{- butènes}}$ = 0,2 bar et $P_{N2}$ = 0,8 bar avec une vitesse spatiale de 2g de n-butènes par g de ferrierite et par heure (2h-1).

Puis la température du réacteur est abaissée à 350°C, qui est la température de la réaction d'isomérisation des n-butènes et la pph maintenue à 2 h-1.

La conversion des n-butènes, les sélectivités et rendements en isobutène sont alors mesurés au bout de 1 heure d'injection de n-butènes et sont reportés dans le tableau 2.

On a pu vérifier des résultats similaires sur un catalyseur constitué de 80 % pds de la zéolite utilisée dans cet exemple et de 20 % pds d'alumine.

**Exemple 4 : Catalyseur 4 non conforme à l'invention**

La même zéolithe ZSM-23 que celle utilisée dans l'exemple 3 est mise oeuvre de la même façon dans un réacteur

à lit fixe et calcinée à 550°C durant 4 heures.

Puis, elle subit un prétraitement qui consiste à réaliser une introduction des n-butènes à isomériser à une température de 150°C durant 2 heures. Les n-butènes sont introduits dilués dans l'azote aux pressions partielles suivantes $P_{n\text{-butènes}}$ = 0,2 bar et $P_{N2}$ = 0,8 bar avec une vitesse spatiale de 2g de n-butènes par g de ferrierite et par heure (2h-1).

Puis la température du réacteur est augmentée à 350°C, qui est la température de la réaction d'isomérisation des n-butènes et la pph maintenue à 2 h-1.

La conversion des n-butènes, les sélectivités et rendements en isobutène sont alors mesurés au bout de 1 heure d'injection de n-butènes et sont reportés dans le tableau 2.

Tableau 2

| | Conversion n-butènes (% poids) | Sélectivité en isobutène (% poids) | Rendement en isobutène (% poids) |
|---|---|---|---|
| Exemple 3 conforme à l'invention | 46 | 40 | 18,4 |
| Exemple 4 non conforme à l'invention | 52 | 20 | 10,4 |

Ce tableau met bien en évidence l'influence du prétraitement selon l'invention de la ZSM-23 sur les sélectivités et les rendements en isobutène qui sont dans ce cas plus élevés que lorsqu'aucun pétraitement n'est appliqué.

**Revendications**

1. Procédé d'isomérisation squelettale d'une charge contenant des hydrocarbures oléfiniques linéaires ayant de 4 à 20 atomes de carbone avec un catalyseur contenant au moins un tamis moléculaire présentant une taille de pores comprise entre 0,4 nm et 0,8 nm, procédé caractérisé en ce que ledit tamis a été préalablement mis au contact d'au moins une molécule hydrocarbonée ayant de 4 à 20 atomes de carbone, à une vitesse spatiale de 0,1-45 h-1, une température de 300-550°C, sous une pression de 0,1-1MPa, pendant 0,5-48h, de façon à déposer du coke dans lesdits pores dudit tamis.

2. Procédé selon la revendication 1, dans lequel la molécule hydrocarbonée possède 4 à 12 atomes de carbone.

3. Procédé selon l'une des revendications précédentes, dans lequel la molécule hydrocarbonée est choisie dans le groupe formé par les monooléfines, les polyoléfines et les alcanes.

4. Procédé selon l'une des revendications précédentes, dans lequel la molécule hydrocarbonée est diluée dans un gaz inerte.

5. Procédé selon l'une des revendications précédentes, dans lequel la température est comprise entre 400-535°C, la pression est comprise entre 0,1 -0,5 MPa, la vitesse spatiale entre 0,5-25 $h^{-1}$ et la durée entre 0,5-24 h.

6. Procédé selon la revendication 1, dans lequel le tamis présente une taille de pore comprise entre 0,4 nm et 0,7 nm.

7. Procédé selon l'une des revendications précédentes, dans lequel le tamis présente un réseau microporeux monodimensionnel.

8. Procédé selon l'une des revendications précédentes, dans lequel le tamis moléculaire est choisi dans le groupe formé par la ferriérite, SAPO-31, SAPO-11 , Theta-1 , EU-1, ZSM-12, ZSM-23, la zéolite oméga, la mordénite, Nu-10, Nu-86 et Nu-87.

**9.** Procédé selon l'une des revendications précédentes, dans lequel le tamis est prétraité avant sa mise en forme dans une matrice.

**10.** Procédé selon l'une des revendications précédentes, dans lequel le tamis est prétraité après sa mise en forme dans une matrice.

**11.** Procédé selon l'une des revendications précédentes, dans lequel le tamis est prétraité à l'extérieur de la zone de réaction.

**12.** Procédé selon l'une des revendications précédentes dans lequel la charge est mise au contact du catalyseur à une température de 150-500°C, une pression de 0,01-1 MPa, une vitesse spatiale de 0,1-10h-1.

**13.** Procédé selon l'une des revendications précédentes appliqué à une charge C4 oléfinique.

**14.** Procédé selon l'une des revendications 1 à 12 appliqué à une charge $C_5$ oléfinique.

**15.** Catalyseur contenant au moins un tamis moléculaire choisi dans le groupe formé par ferrierite, SAPO-31, SAPO-11, Theta-1, EU-1, la zéolite oméga, mordénite, Nu-10, NU-86 et Nu-87, ledit tamis ayant été mis au contct d'au moins une molécule hydrocarbonée ayant de 4 à 20 atomes de carbone, à une vitesse spatiale de 0,1-45$h^{-1}$, à une température de 300-550°C, sous une pression de 0,1-1 MPa, pendant 0,5-48h, de façon à déposer du coke dans lesdits pores dudit tamis.

**16.** Catalyseur selon la revendication 13 dans lequel le tamis est mis au contact d'au moins une molécule hydrocarbonée choisie dans le groupe constitué par les monooléfines, les polyoléfines et les alcanes, ayant 4 à 12 atomes de carbone, à une température de 400-535°C, sous une pression de 0,1-0,5 Mpa, en l'absence d'hydrogène.

**17.** Catalyseur selon l'une des revendications 15 à 16 dans lequel le tamis est prétraité avant sa mise en forme dans une matrice.

**18.** Catalyseur selon l'une des revendications 15 à 16 dans lequel le tamis est prétraité après sa mise en forme dans une matrice.

Office européen des brevets

# RAPPORT DE RECHERCHE EUROPEENNE

Numero de la demande
EP 96 40 0924

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | US-A-4 508 836 (HAAG WERNER O ET AL) 2 Avril 1985<br>* revendications 1,16,17 *<br>* exemple 1 * | 1,2,8, 10, 12-14,16 | B01J29/06<br>C07C5/27 |
| A | US-A-5 234 875 (HAN SCOTT ET AL) 10 Août 1993<br>* revendications 1-10 * | 1-3,5,8 | |
| Y | EP-A-0 111 808 (BAYER AG) 27 Juin 1984<br>* revendications 1,5 * | 1,2,8, 10, 12-14,16 | |
| A | EP-A-0 134 076 (MOBIL OIL CORP) 13 Mars 1985 | | |
| A | US-A-5 158 670 (CODY IAN A ET AL) 27 Octobre 1992 | | |
| | | | **DOMAINES TECHNIQUES RECHERCHES (Int.Cl.6)** |
| | | | B01J<br>C07C |

Le présent rapport a été établi pour toutes les revendications

| Lieu de la recherche | Date d'achèvement de la recherche | Examinateur |
|---|---|---|
| LA HAYE | 22 Juillet 1996 | Thion, M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)